(19) Europäisches Patentamt
European Patent Office
Office européen des brevets

(11) **EP 4 495 158 A1**

(12) **EUROPEAN PATENT APPLICATION**
published in accordance with Art. 153(4) EPC

(43) Date of publication:
**22.01.2025 Bulletin 2025/04**

(21) Application number: **23770600.7**

(22) Date of filing: **08.03.2023**

(51) International Patent Classification (IPC):
***C08G 59/42*** (2006.01)   ***C08J 3/12*** (2006.01)

(52) Cooperative Patent Classification (CPC):
**C08G 59/42; C08J 3/12**

(86) International application number:
**PCT/JP2023/008905**

(87) International publication number:
**WO 2023/176644 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.03.2022  JP 2022043023**

(71) Applicant: **SUMITOMO SEIKA Chemicals Co., Ltd.
Kako-gun, Hyogo 675-0145 (JP)**

(72) Inventor: **NISHIDA Moe
Himeji-shi, Hyogo 672-8076 (JP)**

(74) Representative: **Vossius & Partner
Patentanwälte Rechtsanwälte mbB
Siebertstrasse 3
81675 München (DE)**

(54) **WATER ABSORBENT RESIN PARTICLES AND ABSORBENT ARTICLE**

(57)   The present invention relates to a water-absorbent resin particle containing a crosslinked polymer having, as a monomer unit, at least one ethylenically unsaturated monomer selected from the group consisting of (meth)acrylic acid and a salt thereof, in which a ratio of the (meth)acrylic acid and the salt thereof is 70% to 100% by mol with respect to a total amount of the monomer unit in the crosslinked polymer, a water retention capacity of the water-absorbent resin particle is 40 g/g or more, and a sum of the water retention capacity (g/g) particle and a water absorption amount (g/g) of the water-absorbent resin particle under a load of 4.14 kPa is 70 g/g or more.

**EP 4 495 158 A1**

## Description

### Technical Field

The present invention relates to a water-absorbent resin particle and an absorbent article.

### Background Art

In recent years, water-absorbent resin particles have been widely used in various fields such as sanitary materials (paper diapers, sanitary products, and other materials), agricultural and horticultural materials (water retention agent, soil conditioner, and other materials), and industrial materials (water stop agents, condensation prevention agents, and other materials), and in particular, the water-absorbent resin particles are often used in sanitary materials. As such water-absorbent resin particles, Patent Literature 1 discloses a method for producing a water-absorbent resin, including a step of subjecting a water-soluble ethylenically unsaturated monomer to a reverse phase suspension polymerization method in a petroleum-based hydrocarbon dispersion medium in the presence of a radical polymerization initiator and a dispersion stabilizing agent, in which an ether-ester type nonionic surfactant is used as the dispersion stabilizing agent.

### Citation List

### Patent Literature

[Patent Literature 1] WO2013/031654A1

### Summary of Invention

### Technical Problem

As for absorbent articles such as diapers, a so-called re-wet, in which an absorber absorbs the liquid, followed by returning of the absorbed liquid from the absorber to a subject wearing an absorbent article, may occur. In addition, from the viewpoint of increasing the effective utilization area of the absorber, it is desirable that, after the absorber has absorbed the liquid, the absorbed liquid is easily diffused in the absorber.

An object of the present invention is to provide a water-absorbent resin particle that contributes to an absorber capable of reducing the re-wet after liquid absorption and has the better diffusibility of liquid after liquid absorption.

### Solution to Problem

The present invention provides the following [1] to [5].

[1] A water-absorbent resin particle containing a crosslinked polymer having, as a monomer unit, at least one ethylenically unsaturated monomer selected from the group consisting of (meth)acrylic acid and a salt thereof, in which a ratio of the (meth)acrylic acid and the salt thereof is 70% to 100% by mol with respect to a total amount of the monomer unit in the crosslinked polymer, a water retention capacity of the water-absorbent resin particle in a physiological saline is 40 g/g or more, and a sum of the water retention capacity (g/g) of the water-absorbent resin particle in the physiological saline and a water absorption amount (g/g) of the water-absorbent resin particle under a load of 4.14 kPa is 70 g/g or more.
[2] The water-absorbent resin particle according to [1], in which a water retention capacity of the water-absorbent resin particle in the physiological saline is 50 g/g or more.
[3] The water-absorbent resin particle according to [1] or [2], in which a median particle diameter is 200 $\mu$m or more and 600 $\mu$m or less.
[4] The water-absorbent resin particle according to any one of [1] to [3], in which a content of a residual monomer is 300 ppm by mass or less based on a total amount of the water-absorbent resin particle.
[5] An absorbent article provided with an absorber that contains the water-absorbent resin particle according to any one of [1] to [4].

### Advantageous Effects of Invention

According to the present invention, it is possible to provide the water-absorbent resin particle that contributes to an absorber capable of reducing the re-wet after liquid absorption and has the better diffusibility of liquid after liquid absorption.

**Brief Description of Drawings**

Fig. 1 is a cross-sectional view showing an example of an absorbent article.
Fig. 2 is a schematic view showing a measurement device for a water absorption amount of water-absorbent resin particles under a load.

**Description of Embodiments**

Hereinafter, some embodiments of the present invention will be described in detail. However, the present invention is not limited to the following embodiments.

In the present specification, "acrylic" and "methacrylic" are collectively referred to as "(meth)acrylic". "Acrylate" and "methacrylate" are also referred to as "(meth)acrylate". In a numerical value range described in a stepwise manner in the present specification, an upper limit value or a lower limit value of a numerical value range in a certain step can be optionally combined with an upper limit value or a lower limit value of a numerical value range in another step. In a numerical value range described in the present specification, the upper limit value or the lower limit value of the numerical value range may be replaced with the value shown in the examples. For materials exemplified in the present specification, one kind may be used alone, or two or more kinds may be used in combination. In a case where there are a plurality of substances corresponding to each of components in a composition, a content of each of the components in the composition means the total amount of the plurality of substances present in the composition unless otherwise specified. In the present specification, "physiological saline" is a sodium chloride aqueous solution having a concentration of 0.9% by mass, and the concentration of 0.9% by mass is based on the mass of the physiological saline.

A water-absorbent resin particle according to the present embodiment includes a crosslinked polymer having, as a monomer unit, at least one ethylenically unsaturated monomer selected from the group consisting of (meth)acrylic acid and a salt thereof, in which a ratio of the (meth)acrylic acid and the salt thereof is 70% to 100% by mol with respect to a total amount of a monomer unit in the crosslinked polymer. A water retention capacity of the water-absorbent resin particle in a physiological saline is 40 g/g or more, and a sum of the water retention capacity (g/g) of the water-absorbent resin particle in the physiological saline and a water absorption amount (g/g) of the water-absorbent resin particle under a load of 4.14 kPa is 70 g/g or more.

The water retention capacity of the water-absorbent resin particle in the physiological saline is 40 g/g or more. From the viewpoint of further reducing the re-wet after liquid absorption, the water retention capacity may be 42 g/g or more, 44 g/g or more, 46 g/g or more, 48 g/g or more, 50 g/g or more, 52 g/g or more, 54 g/g or more, 56 g/g or more, 58 g/g or more, 60 g/g or more, or 62 g/g or more. From the viewpoint of making the better diffusibility of liquid after liquid absorption, the upper limit of the water retention capacity may be, for example, 80 g/g or less, 78 g/g or less, 76 g/g or less, 74 g/g or less, 72 g/g or less, 70 g/g or less, 68 g/g or less, 66 g/g or less, or 64 g/g or less. From the viewpoint of further reducing the re-wet after liquid absorption and the viewpoint of making the better diffusibility of liquid after liquid absorption, the water retention capacity may be 40 g/g or more and 80 g/g or less, 46 g/g or more and 74 g/g or less, or 50 g/g or more and 66 g/g or less. The water retention capacity can be measured by a method described in Examples described later.

The water absorption amount under a load of 4.14 kPa (hereinafter, also simply referred to as the "water absorption amount under the load") is a value measured by a water absorption test carried out as the following procedure: the water-absorbent resin particles are arranged on a liquid-permeable sheet (mesh sheet), which is placed on a measurement table having a through-hole; and the water-absorbent resin particles are allowed to absorb a physiological saline supplied from the through-hole without pressure while a load of 4.14 kPa is applied to the water-absorbent resin particles. Usually, an inner diameter of the through-hole is 2 mm. The amount of the water-absorbent resin particles used in the test is 0.100 g, and the water-absorbent resin particles in this amount are uniformly arranged in a cylinder at a position directly above the through-hole, which has an inner diameter of 20 mm serving as the center. The mass [g/g] (the mass per 1 g of the water-absorbent resin particles) of the physiological saline absorbed by the water-absorbent resin particles within 60 minutes from the start of the water absorption is defined as the water absorption amount under the load. Other details of the test conditions will be described in Examples described later.

The sum of the water retention capacity (g/g) of the physiological saline and the water absorption amount under the load (g/g) (hereinafter, also referred to as "the water retention capacity + the water absorption amount under the load") is 70 g/g or more. From the viewpoint of further reducing the re-wet after liquid absorption and the viewpoint of making the better diffusibility of liquid after liquid absorption, the water retention capacity + the water absorption amount under the load may be 72 g/g or more, 74 g/g or more, 76 g/g or more, or 78 g/g or more. The upper limit of the water retention capacity + the water absorption amount under the load may be, for example, 90 g/g or less, 88 g/g or less, 86 g/g or less, 84 g/g or less, 82 g/g or less, or 80 g/g or less. From the viewpoint of further reducing the re-wet after liquid absorption and the viewpoint of making the better diffusibility of liquid after liquid absorption, the water retention capacity + the water absorption amount under the load may be 70 g/g or more and 90 g/g or less, 72 g/g or more and 88 g/g or less, 74 g/g or more and 86 g/g or less, 76 g/g or more and 84 g/g or less, or 78 g/g or more and 82 g/g or less.

The water absorption amount under the load may be 1 g/g or more, 4 g/g or more, 7 g/g or more, 10 g/g or more, 13 g/g or more, or 15 g/g or more. The water absorption amount under the load may be 40 g/g or less, 38 g/g or less, 36 g/g or less, 34 g/g or less, 32 g/g or less, 30 g/g or less, 28 g/g or less, 26 g/g or less, 24 g/g or less, 22 g/g or less, 20 g/g or less, or 18 g/g or less. From the viewpoint of further reducing the re-wet after liquid absorption and the viewpoint of making the better diffusibility of liquid after liquid absorption, the water absorption amount under the load may be, for example, 1 g/g or more and 35 g/g or less, 1 g/g or more and 30 g/g or less, 4 g/g or more and 26 g/g or less, or 10 g/g or more and 22 g/g or less.

From the viewpoint of easily reducing skin irritation, a content of the residual monomer in the water-absorbent resin particle may be 300 ppm by mass or less, 250 ppm by mass or less, 200 ppm by mass or less, 150 ppm by mass or less, 120 ppm by mass or less, 100 ppm by mass or less, 90 ppm by mass or less, 85 ppm by mass or less, 80 ppm by mass or less, 70 ppm by mass or less, or 50 ppm by mass or less based on the total mass of the water-absorbent resin particle. The lower limit of the content of the residual monomer in the water-absorbent resin particle may be, for example, 10 ppm by mass or more, 20 ppm by mass or more, 30 ppm by mass or more, 40 ppm by mass or more, 50 ppm by mass or more, 60 ppm by mass or more, or 70 ppm by mass or more.

The content of the residual monomer in the water-absorbent resin particle can be measured by the following method. 2.0 g of the water-absorbent resin particles are added to a container containing 500 g of the physiological saline, and the mixture is stirred for 60 minutes. After stirring, the water-absorbent resin particles that have been swollen through liquid absorption are separated by filtration. The mass of the monomer present in the filtrate is measured, and the measurement result is converted into a value per mass of the water-absorbent resin particle to obtain a content of the residual monomer. Other details of the test conditions will be described in Examples described later.

The median particle diameter of the water-absorbent resin particles may be, for example, 200 $\mu$m or more and 600 $\mu$m or less, 200 $\mu$m or more and 500 $\mu$m or less, 200 $\mu$m or more and 450 $\mu$m or less, 250 $\mu$m or more and 600 $\mu$m or less, 250 $\mu$m or more and 550 $\mu$m or less, 250 $\mu$m or more and 500 $\mu$m or less, 250 $\mu$m or more and 450 $\mu$m or less, 300 $\mu$m or more and 600 $\mu$m or less, 300 $\mu$m or more and 550 $\mu$m or less, 300 $\mu$m or more and 500 $\mu$m or less, 300 $\mu$m or more and 450 $\mu$m or less, 350 $\mu$m or more and 600 $\mu$m or less, 350 $\mu$m or more and 550 $\mu$m or less, 350 $\mu$m or more and 500 $\mu$m or less, or 350 $\mu$m or more and 450 $\mu$m or less, and from the viewpoint of making the better diffusibility of liquid after liquid absorption, the median particle diameter may be 390 $\mu$m or more and 600 $\mu$m or less, 390 $\mu$m or more and 550 $\mu$m or less, 390 $\mu$m or more and 500 $\mu$m or less, or 390 $\mu$m or more and 450 $\mu$m or less.

The median particle diameter can be measured by the following method. JIS standard sieves are combined in the following order from the upper side: a sieve having an opening of 710 $\mu$m, a sieve having an opening of 600 $\mu$m, a sieve having an opening of 500 $\mu$m, a sieve having an opening of 425 $\mu$m, a sieve having an opening of 300 $\mu$m, a sieve having an opening of 250 $\mu$m, a sieve having an opening of 180 $\mu$m, and a tray. 5 g of the water-absorbent resin particles are placed onto the combined uppermost sieve and classified by using a continuous fully automatic sonic vibration-type sieving measuring apparatus (Robot shifter RPS-205, manufactured by Seishin Enterprise Co., Ltd.). After classification, the mass of the particles remaining on each sieve is calculated as a mass percentage with respect to the total amount to determine a particle size distribution. The relationship between the opening of the sieve and the integrated value of the mass percentage of the particles remaining on the sieve is plotted on logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass is obtained as the median particle diameter. Other details of the test conditions will be described in Examples described later.

A shape of the water-absorbent resin particle is not particularly limited, and may be, for example, a spherical shape (perfectly spherical or nearly perfectly spherical shape), a crushed shape, or a granular shape, and a particle formed of primary particles with each of these shapes in aggregate.

An aspect ratio of the water-absorbent resin particle may be 1.0 to 1.5, 1.0 to 1.4, or 1.0 to 1.3. The water-absorbent resin particle may be spherical, and an aspect ratio of the water-absorbent resin particle may be within the above-mentioned range.

The aspect ratio can be measured by the following method. The water-absorbent resin particles used to measure the aspect ratio are allowed to pass through a 36-mesh (an opening of 425 $\mu$m) standard sieve for JIS Z 8801-1 and adjusted to have a particle diameter retained on a 50-mesh (an opening of 300 $\mu$m) standard sieve. Next, a scanning electron microscope (SEM) photograph of this sample is captured, and 50 particles are optionally selected from the captured image. The maximum length of each selected particle in a longitudinal direction is defined as a long diameter, and the maximum length perpendicular to the major axis of the long diameter is defined as a short diameter to carry out the measurement. An average value of the measured values of the individual particles is calculated to obtain an aspect ratio as a ratio of the long diameter to the short diameter (long diameter/short diameter).

The water-absorbent resin particle according to the present embodiment contains a crosslinked polymer having, as a monomer unit, at least one ethylenically unsaturated monomer selected from the group consisting of (meth)acrylic acid and a salt thereof.

The crosslinked polymer may have another ethylenically unsaturated monomer in addition to (meth)acrylic acid and a salt thereof, as a monomer unit. The other ethylenically unsaturated monomer may be contained in at least one compound selected from the group consisting of 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the other ethylenically unsaturated monomer has an amino group, the amino group may be quaternized.

In a case where the monomer unit in the crosslinked polymer has an acid group, the monomer unit may be used in a polymerization reaction after neutralizing the acid group with an alkaline neutralizing agent. The neutralization degree of the ethylenically unsaturated monomer by the alkaline neutralizing agent may be 10% to 100% by mol, 50% to 90% by mol, or 60% to 80% by mol of the acid group in the ethylenically unsaturated monomer, from the viewpoint of increasing an osmotic pressure of the obtained water-absorbent resin particle, and further enhancing water-absorbent characteristics (such as a water absorption amount). Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or two or more kinds thereof may be used in combination. The alkaline neutralizing agent may be used in the form of an aqueous solution to simplify the neutralization operation.

In the crosslinked polymer, the ratio of (meth)acrylic acid and a salt thereof is 70% to 100% by mol with respect to the total amount of the monomer units in the crosslinked polymer, and may be, for example, 80% to 100% by mol or 90% to 100% by mol.

The crosslinked polymer may be a polymerization reaction product of the ethylenically unsaturated monomers in a reaction mixture containing a radical polymerization initiator and the above-mentioned ethylenically unsaturated monomers.

Examples of the radical polymerization initiator include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamidel, 2,2'-azobis[2-methyl-N-(2-hydroxyethyl)-propionamide], and 4,4'-azobis(4-cyanovaleric acid). The radical polymerization initiator may be used alone, or two or more kinds thereof may be used in combination.

The radical polymerization initiator may contain an azo compound. In a case where the radical polymerization initiator contains the azo compound, it is easy to obtain the water-absorbent resin particles having the water retention capacity and the water retention capacity + the water absorption amount under the load within the above-mentioned numerical range. The radical polymerization initiator may contain a persulfate (for example, potassium persulfate). In a case where the radical polymerization initiator includes the persulfate, it is easy to obtain the water-absorbent resin particle containing the residual monomer, a content of which is within the above-mentioned numerical range. In a case where the radical polymerization initiator contains both the azo compound and the persulfate, it is easy to obtain the water-absorbent resin particle having the water retention capacity and the water retention capacity + the water absorption amount under the load within the above-mentioned numerical range and containing the residual monomers, a content of which is within the above-mentioned numerical range.

The total use amount of the radical polymerization initiator may be, for example, 0.30 mmol or more, 0.35 mmol or more, 0.40 mmol or more, or 0.45 mmol or more and may be 10 mmol or less, 5 mmol or less, 1 mmol or less, 0.8 mmol or less, 0.6 mmol or less, or 0.5 mmol or less, with respect to 1 mol of the ethylenically unsaturated monomer. In a case where the total use amount of the radical polymerization initiator is within the above-mentioned range, it is easy to obtain the water-absorbent resin particle having the water retention capacity and the water retention capacity + the water absorption amount under the load within the above-mentioned numerical range.

In a case where the radical polymerization initiator contains the azo compound, the use amount of the azo compound may be, for example, 0.25 mmol or more, 0.30 mmol or more, or 0.35 mmol or more and may be 5 mmol or less, 1 mmol or less, 0.8 mmol or less, 0.6 mmol or less, or 0.5 mmol or less, with respect to 1 mol of the ethylenically unsaturated monomers. In a case where the use amount of the azo compound is within the above-mentioned range, it is easy to obtain the water-absorbent resin particle having the water retention capacity and the water retention capacity + the water absorption amount under the load within the above-mentioned numerical range.

The ethylenically unsaturated monomer is usually preferably used as an aqueous solution. The concentration of the ethylenically unsaturated monomers in the aqueous solution containing the ethylenically unsaturated monomers (hereinafter, simply referred to as a "monomer aqueous solution") may be 20% by mass or more and a saturated concentration or less, 25% to 70% by mass, or 30% to 55% by mass. Examples of the water used in the aqueous solution include tap water, distilled water, and ion-exchanged water.

The water-absorbent resin particle according to the present embodiment can be produced by, for example, a method including a polymerization step of polymerizing the above-mentioned ethylenically unsaturated monomer in a reaction mixture containing a radical polymerization initiator and the above-mentioned ethylenically unsaturated monomer to obtain a crosslinked polymer, and a surface crosslinking step of subjecting the crosslinked polymer to surface crosslinking with a surface crosslinking agent.

Examples of methods for polymerizing an ethylenically unsaturated monomer include a reverse phase suspension polymerization method, an aqueous solution polymerization method, a bulk polymerization method, a precipitation polymerization method, and other methods. For example, in the reverse phase suspension polymerization method, the polymerization of the ethylenically unsaturated monomer can be performed by dispersing the monomer aqueous solution in a hydrocarbon dispersion medium in the presence of a surfactant and, as necessary, a polymeric dispersant, and using the above-mentioned radical polymerization initiator or the like.

Examples of the surfactant include a nonionic surfactant, an anionic surfactant, and other surfactants. The surfactant may be used alone, or two or more kinds thereof may be used in combination.

Examples of the polymeric dispersant include maleic anhydride-modified polyethylene, maleic anhydride-modified polypropylene, a maleic anhydride-modified polyethylene copolymer, a maleic anhydride-modified polyethylene EPDM (ethylene propylene diene terpolymer), maleic anhydride-modified polybutadiene, a maleic acid anhydride-ethylene copolymer, a maleic acid anhydride-propylene copolymer, a maleic acid anhydride-ethylene-propylene copolymer, a maleic acid anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, and ethyl hydroxyethyl cellulose. The polymeric dispersant may be used alone or may be used in combination of two or more kinds thereof.

The hydrocarbon dispersion medium may contain at least one compound selected from the group consisting of chain aliphatic hydrocarbons having 6 to 8 carbon atoms and alicyclic hydrocarbons having 6 to 8 carbon atoms. The hydrocarbon dispersion medium may be used alone, or two or more kinds thereof may be used in combination.

In order to control the particle diameter of the water-absorbent resin particle, the monomer aqueous solution used for polymerization may contain a thickener such as hydroxyethyl cellulose.

Crosslinking by self-crosslinking may occur during polymerization, but the crosslinking may be induced by using an internal crosslinking agent. The internal crosslinking agent is usually added to a reaction solution during the polymerization reaction. The internal crosslinking agent may be a compound containing two or more functional groups (reactive functional groups) having reactivity with a functional group derived from the ethylenically unsaturated monomer.

Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis(meth)acrylamides such as N,N'-methylenebis(meth)acrylamide; di or tri (meth)acrylic acid esters obtained by reacting a polyepoxide with (meth)acrylic acid; carbamyl di(meth)acrylate esters obtained by reacting a polyisocyanate (such as tolylene diisocyanate and hexamethylene diisocyanate) with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; isocyanate compounds (such as 2,4-tolylene diisocyanate and hexamethylene diisocyanate), and other internal crosslinking agents. These internal crosslinking agents may be used alone or two or more kinds thereof may be used in combination.

The use amount of the internal crosslinking agent may be, for example, 0 mmol or more and 0.05 mmol or less, 0 mmol or more and 0.04 mmol or less, 0 mmol or more and 0.03 mmol or less, 0 mmol or more and 0.02 mmol or less, or 0 mmol or more and 0.01 mmol or less, and may be 0 mmol. As the use amount of the internal crosslinking agent is lower, the water retention capacity of the physiological saline tends to increase. In a case where the use amount of the internal crosslinking agent is within the above-mentioned range, it is easy to obtain the water-absorbent resin particle having the water retention capacity and the water retention capacity + the water absorption amount under the load within the above-mentioned numerical range.

In the surface crosslinking step, crosslinking is obtained by a reaction of a crosslinked polymer on at least a surface layer portion of the water-absorbent resin particle with the surface crosslinking agent. The surface crosslinking agent may be a compound containing two or more functional groups (reactive functional groups) having reactivity with a functional group derived from the ethylenically unsaturated monomer, for example.

Examples of the surface crosslinking agents include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; hydroxyalkylamide compounds such as bis[N,N-di($\beta$-hydroxyethyl)]adipamide; and other surface crosslinking agents. The surface crosslinking agent may be used alone, or two or more kinds thereof may be used in combination.

The use amount of the surface crosslinking agent may be 1.0 mmol or more, 1.5 mmol or more, or 2.0 mmol or more and may be 10 mmol or less, 8.0 mmol or less, 6.0 mmol or less, or 4.0 mmol or less, with respect to 1 mol of the ethylenically unsaturated monomer used for polymerization. In a case where the use amount of the surface crosslinking agent is within the above-mentioned range, it is easy to obtain the water-absorbent resin particle having the water retention capacity and the water retention capacity + the water absorption amount under the load within the above-mentioned numerical range.

A ratio of the amount of the internal crosslinking agent by mole to the amount of the surface crosslinking agent by mole (internal crosslinking agent/surface crosslinking agent) may be 0 or more and 0.05 or less, 0 or more and 0.026 or less, 0 or more and 0.015 or less, or 0 or more and 0.010 or less. In a case where the ratio of the amount of the internal crosslinking agent by mole to the amount of the surface crosslinking agent by mole is within the above-mentioned range, it is easy to obtain the water-absorbent resin particles having the water retention capacity and the water retention capacity + the water absorption amount under the load within the above-mentioned numerical range.

A specific surface area of the water-absorbent resin particles before surface crosslinking may be 0.005 $m^2$/g to 0.025 $m^2$/g, 0.008 $m^2$/g to 0.023 $m^2$/g, or 0.010 $m^2$/g to 0.021 $m^2$/g, 0.01 $m^2$/g to 0.020 $m^2$/g. In a case where the specific surface area is within the above-mentioned range, the uniform crosslinking is likely to be achieved, and as a result, the water retention capacity + the water absorption amount under the load is likely to be increased.

The specific surface area is measured by the following method. The water-absorbent resin particles used to measure the specific surface area are allowed to pass through a 36-mesh (an opening of 425 $\mu$m) standard sieve for JIS Z 8801-1 and adjusted to have a particle diameter retained on a 50-mesh (an opening of 300 $\mu$m) standard sieve. Subsequently, this sample is dried by using a vacuum dryer at a temperature of 100°C for 16 hours under a reduced pressure of about 1 Pa. Thereafter, an adsorption isotherm at -196°C is measured with a high-precision fully automatic gas adsorption apparatus (trade name: BELSORP36, manufactured by BEL JAPAN, INC.), using krypton gas serving as an adsorption gas, and the specific surface area is determined from a multi-point BET plot.

The water-absorbent resin particles may contain a certain amount of water, and may further contain various additional components therein, in addition to polymer particles containing crosslinked polymers. Examples of the additional components include a gel stabilizing agent, a metal chelating agent, and inorganic particles.

The particle size distribution of the water-absorbent resin particles may be adjusted by performing an operation such as particle size adjustment using classification with a sieve, as necessary. For example, a fraction that passed through a sieve having an opening of 850 $\mu$m may be used as a water-absorbent resin particle.

The water-absorbent resin particle of the present embodiment can contribute to an absorber that reduces the re-wet when wearing for a long time, and has the better diffusibility of liquid after liquid absorption. In a case where the absorber has the better diffusibility of liquid after liquid absorption, the utilization of the absorber is improved, which enables wearing for a long time. Furthermore, in a case where the re-wet is reduced when wearing for a long time, it is possible to spend a comfortable time even when wearing for a long time. As a result, it is expected that, for example, the reduction in the frequency of diaper replacement during child care and nursing care can contribute to the reduction in the burden on the consumer, and the reduction in the use amount of diapers can contribute to the reduction in the environmental load.

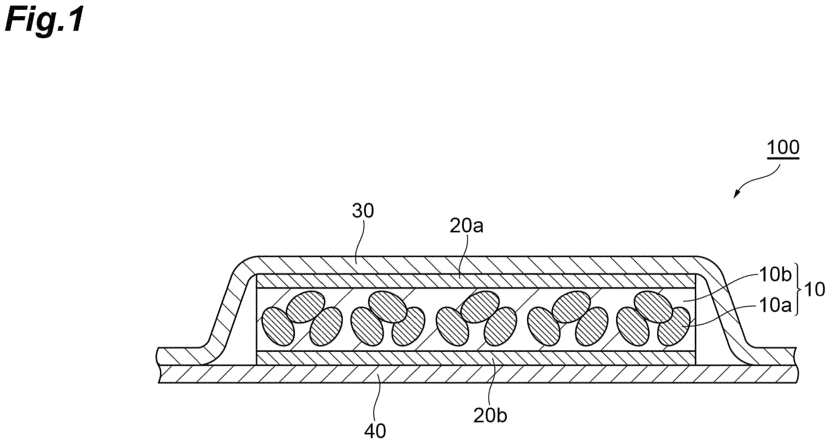

The water-absorbent resin particle is used to form an absorber constituting an absorbent article such as diapers, for example. Fig. 1 is a cross-sectional view showing an example of an absorbent article. An absorbent article 100 shown in Fig. 1 includes a sheet shaped absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that a gap is present between members, but the members may be in close contact with each other without the gap.

The absorber 10 contains water-absorbent resin particles 10a according to the present embodiment mentioned above and a fiber layer 10b containing a fibrous material. The water-absorbent resin particles 10a are dispersed in the fiber layer 10b.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (on a lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, and the like. The core wrap 20a and the core wrap 20b each have, for example, a main surface having the same size as that of the absorber 10.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on the lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 each have, for example, a main surface wider than the main surface of the absorber 10, and outer edges of the liquid permeable sheet 30 and the liquid impermeable sheet 40 each extend around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

According to the present embodiment, it is possible to provide a liquid absorbing method using the water-absorbent resin particle, the absorber, or the absorbent article according to the present embodiment. The liquid absorbing method according to the present embodiment includes a step of bringing liquid to be absorbed into contact with the water-absorbent resin particle, the absorber, or the absorbent article according to the present embodiment.

According to the present embodiment, it is possible to provide a method for reducing the re-wet after liquid absorption in the absorbent article and improving the diffusibility after liquid absorption, the method using the water-absorbent resin particle according to the present embodiment. The method includes a step of adjusting the water retention capacity of the water-absorbent resin particle in the physiological saline, and a sum of the water retention capacity (g/g) of the water-absorbent resin particle in the physiological saline and the water absorption amount of the water-absorbent resin particle under a load of 4.14 kPa. In the adjustment step, the water retention capacity of the water-absorbent resin particle in the physiological saline, and the sum of the water retention capacity (g/g) of the water-absorbent resin particle in the physiological saline and the water absorption amount (g/g) of the water-absorbent resin particle under a load of 4.14 kPa may be adjusted to the above-mentioned numerical range.

According to the present embodiment, it is possible to provide a method for producing a water-absorbent resin particle, the method including a selecting step of selecting a water-absorbent resin particle based on the water retention capacity of the water-absorbent resin particle in the physiological saline, and the sum of the water retention capacity (g/g) of the water-absorbent resin particle in the physiological saline and the water absorption amount (g/g) of the water-absorbent resin particle under a load of 4.14 kPa. In the selecting step, the water retention capacity of the water-absorbent resin particle in the physiological saline, and the sum of the water retention capacity (g/g) of the water-absorbent resin particle in the physiological saline and the water absorption amount (g/g) of the water-absorbent resin particle under a load of 4.14 kPa may be adjusted to the above-mentioned numerical range.

According to the present embodiment, it is possible to provide a method for producing an absorber by using the water-absorbent resin particle obtained by the above-mentioned method for producing a water-absorbent resin particle. The method for producing an absorber according to the present embodiment includes a particle producing step of obtaining a water-absorbent resin particle by the above-mentioned method for producing a water-absorbent resin particle. The method for producing an absorber according to the present embodiment may include a step of mixing the water-absorbent resin particle and a fibrous material after the particle producing step. According to the present embodiment, it is possible to provide a method for producing an absorbent article by using the absorber obtained by the above-mentioned method for producing an absorber. The method for producing an absorbent article according to the present embodiment includes an absorber producing step of obtaining an absorber by the above-mentioned method for producing an absorber. The method for producing an absorbent article of the present embodiment may include a step of obtaining an absorbent article by using the absorber and another constituent member constituting the absorbent article after the absorber producing step. In this step, for example, the absorbent article is obtained by laminating the absorber and the other constituent member constituting the absorbent article with each other.

**Examples**

Hereinafter, the present invention will be more specifically described with reference to examples. However, the present invention is not limited to these examples.

[Production of Water-absorbent Resin Particles]

(Example 1)

A round-bottomed cylindrical separable flask having an inner diameter of 11 cm and an internal volume of 2 L equipped with a reflux cooling device, a dropping funnel, a nitrogen gas introduction tube, and a stirrer was prepared. As the stirrer, a stirrer blade having two stages of four inclined paddle blades with a blade diameter of 5 cm was used. 300 g of n-heptane serving as a hydrocarbon dispersion medium, 0.736 g of maleic acid-modified ethylene-propylene copolymer (Mitsui Chemicals, Inc., Hi-Wax 1105A) serving as a polymeric dispersant, and 0.736 g of sucrose stearic acid ester (HLB: 3, Mitsubishi-Chemical Foods Corporation, Ryoto Sugar Ester S-370) serving as a surfactant were added to the above-mentioned flask, the mixture was stirred at a rotation speed of a stirrer of 400 rpm while being heated to 80°C, and the dispersant and surfactant were dissolved and then cooled to 60°C.

On the other hand, 92.0 g (1.03 mol) of a 80.5% by mass aqueous solution of acrylic acid serving as an ethylenically unsaturated monomer was charged into a beaker having an internal volume of 300 ml, 103.63 g of a 30% by mass aqueous solution of sodium hydroxide was added dropwise while being cooled from the outside to adjust a 75% by mol neutralized product of acrylic acid, and 1.38 g of hydroxyethyl cellulose (HEC AW-15F, SUMITOMO SEIKA CHEMICALS CO., LTD.) serving as a thickener, 0.1105 g (0.407 mmol) of 2,2'-azobis(2-amidinopropane)dihydrochloride serving as an azo-based compound, 0.0184 g (0.068 mmol) of potassium persulfate serving as a peroxide, and 42.71 g of ion-exchanged water were added and dissolved, thereby preparing a monomer aqueous solution.

The prepared monomer aqueous solution was added to the dispersion medium in the above-mentioned separable flask, and the system was sufficiently replaced with nitrogen while stirring. Thereafter, the flask was immersed in a water bath at 70°C and heated, and the mixture was polymerized for 30 minutes.

Thereafter, the flask was immersed in an oil bath set at 125°C while stirring was performed at a rotation speed of the stirrer of 1,000 rpm, and 116.3 g of water was extracted to the outside of the system while n-heptane was refluxed by azeotropic distillation of n-heptane and water. Subsequently, 5.52 g (3.166 mmol) of a 10% by mass aqueous solution of ethylene glycol diglycidyl ether serving as a surface crosslinking agent was added to the flask, and the internal temperature was maintained at 80°C for 2 hours.

Thereafter, the flask was immersed in an oil bath set at 125°C, and n-heptane was evaporated and dried to obtain polymer particles (dried product). These polymer particles were allowed to pass through a sieve having an opening of 850 μm, thereby obtaining 94.3 g of water-absorbent resin particles.

(Example 2)

80.5 g of water-absorbent resin particles were obtained in the same manner as in Example 1, except that 116.6 g of water was extracted to the outside of the system by azeotropic distillation, and the addition amount of a 10% by mass aqueous solution of ethylene glycol diglycidyl ether was changed to 3.68 g (2.110 mmol).

(Example 3)

87.8 g of water-absorbent resin particles were obtained in the same manner as in Example 1, except that 114.8 g of water was extracted to the outside of the system by azeotropic distillation, and the addition amount of a 10% by mass aqueous solution of ethylene glycol diglycidyl ether was changed to 3.68 g (2.110 mmol).

(Example 4)

91.6 g of water-absorbent resin particles were obtained in the same manner as in Example 1, except that 115.1 g of water was extracted to the outside of the system by azeotropic distillation, and the addition amount of a 10% by mass aqueous solution of ethylene glycol diglycidyl ether was changed to 3.68 g (2.110 mmol).

(Example 5)

92.1 g of water-absorbent resin particles were obtained in the same manner as in Example 1, except that 114.5 g of water was extracted to the outside of the system by azeotropic distillation, and the addition amount of a 10% by mass aqueous solution of ethylene glycol diglycidyl ether was changed to 2.76 g (1.583 mmol).

(Comparative Example 1)

A round-bottomed cylindrical separable flask having an inner diameter of 11 cm and an internal volume of 2 L equipped with a reflux cooling device, a dropping funnel, a nitrogen gas introduction tube, and a stirrer was prepared. As the stirrer, a stirrer blade having two stages of four inclined paddle blades with a blade diameter of 5 cm was used. 293 g of n-heptane serving as a hydrocarbon dispersion medium and 0.736 g of a maleic acid-modified ethylene-propylene copolymer (manufactured by Mitsui Chemicals, Inc., Hi-Wax 1105A) serving as a polymeric dispersant were added to the above-mentioned flask, and the mixture was heated to 80°C while being stirred at a rotation speed of the stirrer of 300 rpm, thereby dissolving the dispersant in n-heptane. The formed solution was cooled to 50°C.

92.0 g (1.03 mol) of a 80.5% by mass aqueous solution of acrylic acid serving as a water-soluble ethylenically unsaturated monomer was put into a beaker having an internal volume of 300 mL, and 103.63 g of a 30% by mass aqueous solution of sodium hydroxide was added dropwise while being cooled from the outside, thereby adjusting a 75% by mol neutralized product of acrylic acid. Thereafter, 0.0736 g (0.272 mmol) of potassium persulfate serving as a peroxide, 0.00460 g (0.0264 mmol) of ethylene glycol diglycidyl ether serving as an internal crosslinking agent, and 44.16 g of ion-exchanged water were added and dissolved to prepare a first stage aqueous solution.

The first stage aqueous solution was added to a n-heptane solution containing the dispersant in the above-mentioned flask, and the formed reaction solution was stirred for 10 minutes. Separately, 0.736 g of sucrose stearic acid ester (Mitsubishi-Chemical Foods Corporation, Ryoto Sugar Ester S-370, HLB: 3) serving as a surfactant was dissolved in 6.62 g of n-heptane to prepare a surfactant solution. The surfactant solution was added into the above-mentioned flask, and the inside of the system was sufficiently replaced with nitrogen while the reaction solution was stirred at a rotation speed of the stirrer of 500 rpm. Thereafter, the above-mentioned flask was immersed in a water bath at 70°C to increase the temperature of the reaction solution, and the polymerization reaction was advanced for 60 minutes to obtain a first stage polymerization slurry solution.

128.8 g (1.44 mol) of a 80.5% by mass aqueous solution of acrylic acid was put into another beaker having an internal volume of 500 mL, and 145.1 g of a 30% by mass aqueous solution of sodium hydroxide was added dropwise thereto while being cooled from the outside, thereby adjusting a 75% by mol neutralized product of acrylic acid. Thereafter, 0.0902 g (0.379 mmol) of potassium persulfate serving as a peroxide and 16.02 g of ion-exchanged water were added and dissolved to prepare a second stage aqueous solution.

The first stage polymerization slurry solution in the above-mentioned flask was cooled to 23°C while being stirred at a rotation speed of the stirrer of 1,000 rpm, and the total amount of the second stage aqueous solution was added thereto. After the inside of the flask was replaced with nitrogen for 30 minutes, the flask was immersed in a water bath at 70°C again to increase the temperature of the reaction solution, and a second stage polymerization reaction occurred for 60 minutes to obtain a hydrogel-like polymer.

Thereafter, the above-mentioned flask was immersed in an oil bath set at 125°C, and 265.2 g of water was extracted to the outside of the system while n-heptane was refluxed by azeotropic distillation of n-heptane and water. Subsequently, 5.52 g (0.633 mmol) of a 2% by mass aqueous solution of ethylene glycol diglycidyl ether serving as a surface crosslinking agent was added to the flask, and the internal temperature was maintained at 83°C for 2 hours.

Thereafter, the flask was immersed in an oil bath set at 125°C, and n-heptane was evaporated and dried to obtain polymer particles (dried product). These polymer particles were allowed to pass through a sieve having an opening of 850 $\mu$m, thereby obtaining 227.5 g of water-absorbent resin particles.

(Comparative Example 2)

A round-bottomed cylindrical separable flask having an inner diameter of 11 cm and an internal volume of 2 L equipped with a reflux cooling device, a dropping funnel, a nitrogen gas introduction tube, and a stirrer was prepared. As the stirrer, a stirrer blade having two stages of four inclined paddle blades with a blade diameter of 5 cm was used. 293 g of n-heptane serving as a hydrocarbon dispersion medium and 0.736 g of a maleic acid-modified ethylene-propylene copolymer (manufactured by Mitsui Chemicals, Inc., Hi-Wax 1105A) serving as a polymeric dispersant were added to the above-mentioned flask, and the mixture was heated to 80°C while being stirred at a rotation speed of the stirrer of 300 rpm, thereby dissolving the dispersant in n-heptane. The formed solution was cooled to 50°C.

92.0 g (1.03 mol) of a 80.5% by mass aqueous solution of acrylic acid serving as a water-soluble ethylenically unsaturated monomer was put into a beaker having an internal volume of 300 mL, and 103.6 g of a 30% by mass aqueous solution of sodium hydroxide was added dropwise thereto while being cooled from the outside, thereby adjusting a 75% by mol neutralized product of acrylic acid. Thereafter, 0.092 g of hydroxyethyl cellulose (SUMITOMO SEIKA CHEMICALS CO., LTD., HEC AW-15F) serving as a thickener, 0.092 g (0.339 mmol) of 2,2'-azobis(2-amidinopropane)dihydrochloride serving as an azo-based compound, 0.018 g (0.068 mmol) of potassium persulfate serving as a peroxide, 0.1012 g (0.026 mmol) of ethylene glycol diglycidyl ether serving as an internal crosslinking agent, and 44.12 g of ion-exchanged water were added and dissolved to prepare a first stage aqueous solution.

The first stage aqueous solution was added to a n-heptane solution containing the dispersant in the above-mentioned flask, and the formed reaction solution was stirred for 10 minutes. Separately, 0.736 g of sucrose stearic acid ester (Mitsubishi-Chemical Foods Corporation, Ryoto Sugar Ester S-370, HLB: 3) serving as a surfactant was dissolved in 6.62 g of n-heptane to prepare a surfactant solution. The surfactant solution was added into the above-mentioned flask, and the inside of the system was sufficiently replaced with nitrogen while the reaction solution was stirred at a rotation speed of the stirrer of 550 rpm. Thereafter, the above-mentioned flask was immersed in a water bath at 70°C to increase the temperature of the reaction solution, and the polymerization reaction was advanced for 60 minutes to obtain a first stage polymerization slurry solution.

128.8 g (1.44 mol) of a 80.5% by mass aqueous solution of acrylic acid was put into another beaker having an internal volume of 500 mL, and 145.1 g of a 30% by mass aqueous solution of sodium hydroxide was added dropwise thereto while being cooled from the outside, thereby adjusting a 75% by mol neutralized product of acrylic acid. 0.129 g (0.475 mmol) of 2,2'-azobis(2-amidinopropane)dihydrochloride serving as a radical polymerization initiator, 0.026 g (0.095 mmol) of potassium persulfate, 0.0116 g (0.067 mmol) of ethylene glycol diglycidyl ether serving as an internal crosslinking agent, and 15.98 g of ion-exchanged water were added to the beaker containing the aqueous solution of acrylic acid that has been adjusted to dissolve these, thereby preparing a second stage aqueous solution.

The first stage polymerization slurry solution in the above-mentioned flask was cooled to 25°C while being stirred at a rotation speed of the stirrer of 1,000 rpm, and the total amount of the second stage aqueous solution was added thereto. After the inside of the flask was replaced with nitrogen for 30 minutes, the flask was immersed in a water bath at 70°C again to increase the temperature of the reaction solution, and a second stage polymerization reaction occurred for 60 minutes to obtain a hydrogel-like polymer.

Thereafter, the above-mentioned flask was immersed in an oil bath set at 125°C, and thereafter, the flask was immersed in an oil bath set at 125°C, and 214.5 g of water was extracted to the outside of the system while n-heptane was refluxed by azeotropic distillation of n-heptane and water. Subsequently, 4.42 g (0.507 mmol) of a 2% by mass aqueous solution of ethylene glycol diglycidyl ether serving as a surface crosslinking agent was added to the flask, and the internal temperature was maintained at 83°C for 2 hours.

Thereafter, the flask was immersed in an oil bath set at 125°C, and n-heptane was removed by drying to obtain polymer particles (dried product). These polymer particles were allowed to pass through a sieve having an opening of 850 μm, and then mixed with polymer particles based on the mass of the polymer particles to obtain 236.6 g of water-absorbent resin particles.

[Evaluation of Water-absorbent Resin Particles]

(Water Retention Capacity)

The water retention capacity (room temperature, 25°C ± 2°C) of the water-absorbent resin particles in a physiological saline was measured by the following procedure. First, a cotton bag (cotton broadcloth No. 60, a width of 100 mm × a length of 200 mm) into which 2.0 g of the weighted water-absorbent resin particles had been weighed was placed in a beaker having the internal volume of 500 mL. After 500 g of the physiological saline was poured into the cotton bag containing the water-absorbent resin particles at one time so that lumps could not be produced, the upper part of the cotton bag was bound with a rubber band, and the cotton bag was left to stand for 30 minutes to swell the water-absorbent resin particles. The cotton bag after an elapse of 30 minutes was dehydrated for 1 minute using a dehydrator (manufactured by KOKUSAN Co., Ltd., product number: H-122) which had been set to have the centrifugal force of 167 G, and a mass Wa [g] of the cotton bag containing the swollen gel after dehydration was then measured. The same operation was performed without addition of the water-absorbent resin particles, an empty mass Wb [g] at the time when the cotton bag was wet was measured, and the water retention capacity of the water-absorbent resin particles in the physiological saline was calculated from the following expression.

$$\text{Water retention capacity } [g/g] = (Wa - Wb)/2.0$$

(Water Absorption Amount under Load)

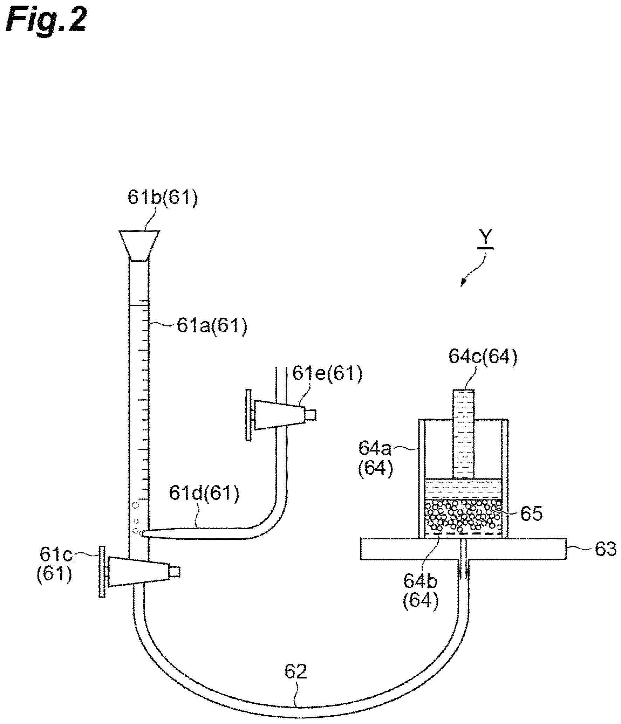

The water absorption amount of the water-absorbent resin particles in the physiological saline under a load (under pressurization) (room temperature) was measured by using a measurement device Y shown in Fig. 2. The measurement device Y is formed of a burette unit 61, a conduit 62, a measurement table 63, and a measurement unit 64 placed on the measurement table 63. The burette unit 61 has a burette 61a extending in a vertical direction, a rubber stopper 61b disposed on the upper end of the burette 61a, a cock 61c disposed on the lower end of the burette 61a, an air introduction tube 61d having one end that extends into the burette 61a in the vicinity of the cock 61c, and a cock 61e disposed on the other end side of the air introduction tube 61d. The conduit 62 is attached between the burette unit 61 and the measurement table 63. The inner diameter of the conduit 62 is 6 mm. At the central portion of the measurement table 63, a hole having a diameter of 2 mm is formed, and the conduit 62 is connected thereto. The measurement unit 64 has a cylinder 64a (made of acrylic resin (plexiglas)), a nylon mesh 64b adhered to the bottom of the cylinder 64a, and a weight 64c. The inner diameter of the cylinder 64a is 20 mm. The opening of the nylon mesh 64b is 75 $\mu$m (200 mesh). At the time of measurement, water-absorbent resin particles 65 to be measured are uniformly scattered on the nylon mesh 64b.

The weight 64c has a diameter of 19 mm and a mass of 119.6 g. The weight 64c is placed over the water-absorbent resin particles 65, and can apply a load of 4.14 kPa to the water-absorbent resin particles 65.

After 0.100 g of the water-absorbent resin particles 65 were put in the cylinder 64a of the measurement device Y, the weight 64c was placed, and the measurement was started. The amount of reduction in the water level of the physiological saline inside the burette 61a corresponds to the amount of the physiological saline absorbed by the water-absorbent resin particles 65 because the same volume of air as that of the physiological saline absorbed by the water-absorbent resin particles 65 is quickly and smoothly supplied to the inside of the burette 61a from the air introduction tube.

A scale of the burette 61a is engraved from top to bottom in increments of 0 mL to 0.5 mL; as the water level of the physiological saline, a scale Va of the burette 61a before the start of water absorption and a scale Vb of the burette 61a in 60 minutes from the start of the water absorption are read; and the water absorption amount under the load was calculated by the following expression.

Water absorption amount under load [g/g] = (Vb - Va) [ml] $\times$ 1.0028 [g/ml] (specific gravity of 0.9% by weight NaCl)/ 0.1 [g]

(Median Particle Diameter)

The median particle diameter of the particles was measured according to the following procedure in an environment of room temperature (25 $\pm$ 2°C) and a humidity of 50 $\pm$ 10%. A continuous fully automatic sonic vibration-type sieving measuring apparatus (Robot shifter RPS-205, manufactured by Seishin Enterprise Co., Ltd.) was used to measure the particle size distribution of 5 g of the water-absorbent resin particles was measured with sieves having openings of 710 $\mu$m, 600 $\mu$m, 500 $\mu$m, 425 $\mu$m, 300 $\mu$m, 250 $\mu$m, and 180 $\mu$m and a tray in accordance with JIS standards. The relationship between the opening of the sieve and the integrated value of the mass percentage of the particles remaining on the sieve was plotted on logarithmic probability paper by integrating in the order from the one having the largest particle diameter on the sieve with respect to this particle size distribution. The plots on the probability paper are connected with a straight line to obtain the particle diameter corresponding to a cumulative mass percentage of 50% by mass as the median particle diameter.

(Content of Residual Monomer)

500 g of the physiological saline was put into a 500 mL beaker, and 2.0 g of water-absorbent resin particles was added thereto and stirred for 60 minutes. The contents of the beaker were allowed to pass through a JIS standard sieve having an opening of 75 $\mu$m, followed by the filtration through a syringe filter (DISMIC-25HP045AN manufactured by ADVANTEC CO., LTD.) to separate a gel-like resin with the physiological saline absorbed from the physiological saline as a filtrate. The amount of residual monomers dissolved in the obtained filtrate was measured by high performance liquid chromatography. Here, the residual monomers to be measured are acrylic acid and an alkali metal salt thereof. These measured values were converted into values per mass of the water-absorbent resin particles and defined as a residual monomer content (mass ppm).

(Aspect Ratio)

The water-absorbent resin particles used to measure the aspect ratio were allowed to pass through a 36-mesh (an opening of 425 $\mu$m) standard sieve for JIS Z 8801-1 and adjusted to have a particle diameter retained on a 50-mesh (an opening of 300 $\mu$m) standard sieve. Subsequently, a scanning electron microscope photograph (SEM) of this sample was taken. Fifty particles were randomly selected from the photograph, and the maximum length of each particle in a longitudinal direction was defined as a long diameter, and the maximum length perpendicular to the major axis of the long diameter was defined as a short diameter to carry out the measurement. An average value of the measured values of individual particles was calculated, and an aspect ratio (ratio of long diameter to short diameter) of the resin particles was calculated.

(Specific Surface Area)

The specific surface area is measured by the following method. The water-absorbent resin particles used to measure the specific surface area were allowed to pass through a 36-mesh (an opening of 425 $\mu$m) standard sieve for JIS Z 8801-1 and adjusted to have a particle diameter retained on a 50-mesh (an opening of 300 $\mu$m) standard sieve. Subsequently, this sample was dried by using a vacuum dryer at a temperature of 100°C for 16 hours under a reduced pressure of about 1 Pa. Thereafter, an adsorption isotherm at -196°C was measured with a high-precision fully automatic gas adsorption apparatus (trade name: BELSORP36, manufactured by BEL JAPAN, INC.), using krypton gas serving as an adsorption gas, and the specific surface area was determined from a multi-point BET plot.

[Performance Evaluation of Absorber]

(Preparation of Artificial Urine)

A solution was prepared by blending and dissolving components in ion-exchanged water so that inorganic salts were present as shown below, and a small amount of Blue No. 1 was further blended therein to prepare artificial urine.

Composition of artificial urine

- · Deionized water: 5919.6 g
- · NaCl 60.0g
- · CaCl$_2$· H$_2$O 1.8 g
- · MgCl$_2$· 6H$_2$O 3.6 g
- · Food Blue No. 1 (for coloration)
- · 1%-Triton X-100 15.0g

(Production of Absorbent Article for Evaluation)

A sheet-shaped absorber having a size of 40 cm × 12 cm was produced by uniformly mixing 10 g of the water-absorbent resin particles and 8.0 g of pulverized pulp by air papermaking with an air flow type mixer (Padformer manufactured by O-tec Co., Ltd.). Subsequently, a load of 196 kPa was applied to the entire body for 30 seconds and pressed in a state of the upper and lower sides of the absorber being sandwiched between two sheets of tissue paper having the same size as that of the absorber and a basis weight of 16 g/m$^2$ to form a laminate. Furthermore, an air-through-type porous liquid permeable sheet having the same size as that of the above-mentioned absorber and a basis weight of 22 g/m$^2$, which was made of polyethylene-polypropylene, was disposed on the upper surface of the laminate to obtain an absorbent article for evaluation.

(Evaluation of Amount of Re-wet and Diffusion Distance)

The above-mentioned absorbent article for evaluation was placed on a horizontal table so that a surface provided with the liquid permeable sheet corresponded to the upper surface, and a 100 mL liquid injection cylinder (mass of 60 g) having an inlet with an inner diameter of 3 cm was placed on the central portion of the absorbent article for evaluation. 80 ml of artificial urine was injected into the above-mentioned cylinder at one time. The cylinder was removed from the absorbent article, and the absorbent article was left to stand as it was. The same operation was performed after 15 minutes (second time), 30 minutes (third time), 45 minutes (fourth time), and 60 minutes (fifth time) from the start of the first artificial urine injection at the same position as in the first time using a measurement apparatus. After an elapse of 30 minutes from the fifth injection, 40 sheets of 10 cm square filter paper whose mass (Wd (g)) was measured preliminarily were placed near the injection position of the artificial urine on the absorbent article, and a weight having a bottom surface of 10 cm × 10 cm and a mass of 5 kg was placed thereon. After the application of load for 5 minutes, a mass of the filter paper (We (g)) was measured, and the increased mass was defined as a re-wet amount (g). The test of the re-wet amount was performed in a room adjusted to 25°C and a humidity of 50% (RH).

$$\text{Re-wet amount (g)} = \text{We - Wd}$$

After the measurement of the re-wet, the porous liquid permeable sheet was removed from the absorbent article for evaluation, and a distance (maximum value: 40 cm) where the liquid diffused by passing through the center part of the absorber along the longitudinal direction of the absorber was measured and recorded as the evaluation result of diffusibility. The diffusion length was measured in a unit of 0.5 cm. Table 1 shows the evaluation results of the re-wet amount and the diffusion distance.

[Table 1]

| Examples Comparative Examples | Water retention capacity [g/g] | Water absorption amount under load [g/g] | water retention capacity + water absorption amount under load [g/g] | Residual monomer [mass ppm] | Median particle diameter [$\mu$m] | Aspect ratio | Specific surface area [m$^2$/g] | Performance of absorber | |
|---|---|---|---|---|---|---|---|---|---|
| | | | | | | | | Re-wet amount [g] | Diffusion distance [cm] |
| Example 1 | 72 | 3 | 75 | 78 | 330 | 1.0 | 0.015 | 13.4 | 37.0 |
| Example 2 | 64 | 16 | 80 | 74 | 413 | 1.0 | 0.014 | 5.4 | 40.0 |
| Example 3 | 53 | 26 | 79 | 80 | 408 | 1.0 | 0.018 | 6.5 | 40.0 |
| Example 4 | 48 | 28 | 76 | 75 | 384 | 1.0 | 0.016 | 12.1 | 38.0 |
| Example 5 | 44 | 28 | 72 | 82 | 361 | 1.0 | 0.015 | 27.8 | 40.0 |
| Comparative Example 1 | 55 | 8 | 63 | 60 | 385 | 1.8 | 0.040 | 52.8 | 29.0 |
| Comparative Example 2 | 38 | 27 | 65 | 110 | 361 | 1.6 | 0.026 | 61.2 | 40.0 |

It was shown that the water-absorbent resin particles in Examples contributed to the absorber that was capable of reducing the re-wet after liquid absorption and had the better diffusibility of liquid after liquid absorption.

**Reference Signs List**

10: Absorber

10a, 65: water-absorbent resin particle

10b: fiber layer

20a, 20b: core wrap

30: liquid permeable sheet

40: liquid impermeable sheet

61: burette unit

61a: burette

61b: rubber stopper

61c, 61e: cock

61d: air introduction tube

62: conduit

63: measurement table

64: measurement unit

64a: cylinder

64b: nylon mesh

64c: weight

100: absorbent article

Y: measurement device

**Claims**

1. A water-absorbent resin particle comprising:

   a crosslinked polymer having, as a monomer unit, at least one ethylenically unsaturated monomer selected from the group consisting of (meth)acrylic acid and a salt thereof,
   wherein a ratio of the (meth)acrylic acid and the salt thereof is 70% to 100% by mol with respect to a total amount of the monomer unit in the crosslinked polymer,
   a water retention capacity of the water-absorbent resin particle in a physiological saline is 40 g/g or more, and
   a sum of the water retention capacity (g/g) of the water-absorbent resin particle in the physiological saline and a water absorption amount (g/g) of the water-absorbent resin particle under a load of 4.14 kPa is 70 g/g or more.

2. The water-absorbent resin particle according to claim 1,
   wherein the water retention capacity of the water-absorbent resin particle in the physiological saline is 50 g/g or more.

3. The water-absorbent resin particle according to claim 1,
   wherein a median particle diameter is 200 μm or more and 600 μm or less.

4. The water-absorbent resin particle according to claim 1,
   wherein a content of a residual monomer is 300 ppm by mass or less based on a total amount of the water-absorbent resin particle.

5. An absorbent article comprising:
   an absorber comprising the water-absorbent resin particle according to any one of claims 1 to 4.

*Fig.1*

# Fig.2

## INTERNATIONAL SEARCH REPORT

| | International application No. |
|---|---|
| | **PCT/JP2023/008905** |

**A. CLASSIFICATION OF SUBJECT MATTER**

*C08G 59/42*(2006.01)i; *C08J 3/12*(2006.01)i
FI:   C08G59/42; C08J3/12

According to International Patent Classification (IPC) or to both national classification and IPC

**B. FIELDS SEARCHED**

Minimum documentation searched (classification system followed by classification symbols)

C08G59/42; C08J3/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2016-028117 A (SUMITOMO SEIKA CHEMICALS) 25 February 2016 (2016-02-25) claims 1-8, paragraphs [0084], [0094]-[0158], example 5 | 1-5 |
| X | WO 2020/184387 A1 (SUMITOMO SEIKA CHEMICALS) 17 September 2020 (2020-09-17) paragraphs [0092]-[0125], comparative example 6 | 1-5 |
| X | JP 2020-093243 A (SUMITOMO SEIKA CHEMICALS) 18 June 2020 (2020-06-18) claims 1-5, paragraphs [0086]-[0132], example 3 | 1-5 |
| X | JP 2012-236898 A (SUMITOMO SEIKA CHEMICALS) 06 December 2012 (2012-12-06) claims 1-9, paragraphs [0083]-[0102], examples 1-4, comparative examples 1-3 | 1-5 |
| A | JP 2016-028131 A (SUMITOMO SEIKA CHEMICALS) 25 February 2016 (2016-02-25) claim 1, paragraph [0129] | 1–5 |

☐ Further documents are listed in the continuation of Box C.      ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **16 May 2023** | **30 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

| International application No. |
| --- |
| **PCT/JP2023/008905** |

| Patent document cited in search report | | | Publication date (day/month/year) | Patent family member(s) | Publication date (day/month/year) |
| --- | --- | --- | --- | --- | --- |
| JP | 2016-028117 | A | 25 February 2016 | US 2017/0107313 A1 <br> claims 1-8, paragraphs [0088], [0098]-[0157], example 5 <br> EP 2993191 A1 <br> KR 10-2016-0017649 A <br> CN 105517660 A | |
| WO | 2020/184387 | A1 | 17 September 2020 | US 2022/0143576 A1 <br> paragraphs [0108]-[0149], comparative example 6 <br> EP 3936536 A1 <br> CN 113544168 A <br> KR 10-2021-0137069 A | |
| JP | 2020-093243 | A | 18 June 2020 | US 2022/0023830 A1 <br> claims 1-5, paragraphs [0090]-[0142], example 3 <br> EP 3896115 A1 <br> CN 113166435 A <br> KR 10-2021-0101249 A | |
| JP | 2012-236898 | A | 06 December 2012 | (Family: none) | |
| JP | 2016-028131 | A | 25 February 2016 | US 2017/0210831 A1 <br> claim 1, paragraph [0130] <br> EP 3153528 A1 <br> KR 10-2016-0142416 A <br> CN 106471013 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013031654 A1